Europäisches Patentamt

European Patent Office

Office européen des brevets

(11) Veröffentlichungsnummer: **0 430 340 A2**

## (12) EUROPÄISCHE PATENTANMELDUNG

(21) Anmeldenummer: 90203051.9

(51) Int. Cl.⁵: **A61B 5/00**

(22) Anmeldetag: **19.11.90**

(30) Priorität: 23.11.89 DE 3938759

(43) Veröffentlichungstag der Anmeldung:
05.06.91 Patentblatt 91/23

(84) Benannte Vertragsstaaten:
DE FR GB Patentblatt

(71) Anmelder: **Philips Patentverwaltung GmbH**
**Wendenstrasse 35 Postfach 10 51 49**
**W-2000 Hamburg 1(DE)**

(84) **DE**

Anmelder: **N.V. Philips' Gloeilampenfabrieken**
**Groenewoudseweg 1**
**NL-5621 BA Eindhoven(NL)**

(84) **FR GB**

(72) Erfinder: **Martens, Gerhard, Dr.**
**Breslauer Strasse 40**
**W-2359 Henstedt-Ulzburg(DE)**
Erfinder: **Kordts, Jürgen**
**Schinkelring 63a**
**W-2000 Norderstedt(DE)**
Erfinder: **Helzel, Thomas**
**Oersdorfer Stieg 18**
**W-2358 Kaltenkirchen(DE)**

(74) Vertreter: **Kupfermann, Fritz-Joachim,**
**Dipl.-Ing. et al**
**Philips Patentverwaltung GmbH**
**Wendenstrasse 35 Postfach 10 51 49**
**W-2000 Hamburg 1(DE)**

(54) **Nichtinvasive Oximeteranordung.**

(57) Die Erfindung betrifft eine nichtinvasive Oximeteranordnung mit einem klammerartigen Sensor (11) für beispielsweise einen Finger, einer selbstkalibrierenden Steuereinheit (12) zur Erfassung und Verarbeitung von zwei elektromagnetischen Wellen vorbestimmter Wellenlänge und einer zwischengeschalteten Übertragungsstrecke (13) zum Einsatz in stark elektromagnetisch beeinflußter Umgebung, bei gleichzeitiger Beseitigung von Stör- und Fehlerquellen, wie Bewegungsfehler, Koppelfaktoren des Sensors am Finger, etc. Die Oximeteranordnung weist dazu einen Sensor (11) und eine Übertragungsstrecke (13) aus nicht metallischem Material auf, ferner einen Sensor (11) mit einer Federvorrichtung und mindestens einer Mulde (22) mit einem einer Fingerkuppe angeformten Endabschnitt (23), in dem Lichtleiterabschnitte (24, 25) enden und lösbar zur bewegungsunempfindlichen verbindung des Fingers mit letztlich den sensorseitigen Enden der Übertragungsstrecke (13) Verbunden sind, die zwei Lichtwellenleiter (31, 32) mit unterschiedlichem Querschnitt hat. Die Steuereinheit (12) weist darüber hinaus einen Sender (15), einen Empfänger (16) und eine Recheneinheit (17) zur Ermittlung des Sauerstoffgehalts des Blutes aus der relativen Größe des pulsschlagabhängigen Modulationsgrades der vom Finger reflektierten zuvor unterschiedlich kodierten, den spektralen Fenstern der Übertraungsstrecke (13) entsprechenden elektromagnetischen Wellen auf.

EP 0 430 340 A2

FIG.1

# NICHTINVASIVE OXIMETERANORDNUNG

Die Erfindung betrifft eine nichtinvasive Oximeteranordnung mit einem klammerartigen Sensor für beispielsweise einen Finger, einer selbstkalibrierenden Steuereinheit zur Erzeugung und Verarbeitung von zwei elektromagnetischen Wellen bestimmter Wellenlänge und einer zwischengeschalteten Übertragungsstrecke.

Derartige Anordnungen Werden im allgemeinen benutzt um die Sauerstoffsättigung im menschlichen Blut zu überwachen, beispielsweise als Kontrolle der Lebensfunktionen narkosierter Patienten. Mit Hilfe von optischen Streumessungen an oberflächennah durchflossenen Körperteilen, wie z.B. Ohrläppchen oder Fingerspitzen, erhält man ein Maß für den Absorptionskoffizienten des Blutes. Die Größe des Absorptionskoeffizienten von Blut ist bei rotem Licht stark vom Sauerstoffgehalt abhängig und bei Licht im nahen Infrarotbereich davon nahezu unabhängig. Durch Messung des Intensitätsverhältnisses des Lichtes beider Wellenlängenbereiche kann ein Maß für die Sauerstoffsättigung des Blutes erhalten Werden.

Bei der Anwendung dieses Meßprinzips haben sich jedoch erhebliche Stör- und Fehlerquellen gezeigt. So hängt das optisch meßbare Signal beißpielsweise stark von den Koppelfaktoren des optischen Senders und des Empfängers zur Hautoberfläche ab, so daß die Anbringung des Sensors beispielsweise an einem Finger überaus bewegungsempfindlich ist. Ferner ist das optisch meßbare Signal von der Größe des optisch erfaßten Blutvolumens im betreffenden Körpergewebe abhängig. Für eine genaue quantitative Erfassung des Sauerstoffgehalts ist weiterhin die Kenntnis des Verhältnisses der Gewebeabsorptionen, also ohne Blut für beide Wellenbereiche notwendig.

Aus der Gebrauchsanweisung "Nellcor Pulsoximeter, Modell N-100E, S. 27, 28, 39 und 40", dies ist eine deutsche Übersetzung der Dräger Werke AG, Lübeck, Sept. 1986, des "USERS MANUEL" A 2044, REV A, der Firma Nellcor Incorporated, Hayward, Californien", ist eine Oximeteranordnung bekannt, die aus einem klammerartigen Sensor, einer elektrischen Übertragungsstrecke und einem Signalverarbeitungsgerät mit einem Mikroprozessor und Anzeigen besteht. Der klammerartige Sensor weist Mulden auf, die einen Finger teilweise aufnehmen. Die Mulden sind aus elastischem Kunststoffmaterial und weisen je ein Fenster aus transparentem Material auf, hinter dem im oberen Klammerteil eine Doppel-LED und im unteren Klammerteil eine großflächige Fotodiode angeordnet ist. Die LED's senden rotes Licht im Bereich von 660 nm und Licht im Infrarotbereich von 920 nm in das durchblutete Gewebe. Der Anteil des Lichts, der vom Gewebe und dem nicht pulsierenden Blut absorbiert wird, ist für beide Wellenlängen unterschiedlich. Dieser Anteil verändert sich aber nicht wesentlich während eines Pulsschlages. Das durchgelassene restliche Licht wird von der Fotodiode aufgefangen. Wenn momentan der pulsierende Anteil verschwunden ist, stellt diese Intensität die Anfangs- oder Bezugsintensität für den pulsierenden Anteil der Absorption dar. Ist die pulsierende Komponente vorhanden, dann wird das Licht, das von der Fotodiode bei beiden Wellenlängen empfangen wird, weiter um die Menge reduziert, die vom pulsierenden Blut bei der bestimmten Wellenlänge absorbiert wird. Mit dieser Intensität bei beiden Wellenlängen, zusammen mit den Anfangsintensitäten bei beiden Wellenlängen wird in der Steuereinheit das Verhältnis des oxigenierten Blutes zum Gesamtblut bestimmt. Diese bekannte Oximeteranordnung ist aufwendig und nicht geeignet, die o.g. Probleme völlig zu lösen, wie z.B. in stark elektromagnetisch gestörter Umgebung störungsfrei und patientensicher zu arbeiten. Ferner besteht eine Bewegungsempfindlichkeit hinsichtlich des Koppelfaktors und es können Fremdlichteinflüsse das Meßergebnis verfälschen, wie auch die gegeneinander bewegliche Anordnung des optischen Senders gegenüber dem optischen Empfänger.

Insbesondere im Hinblick auf den bedeutenden klinischen Einsatz von Therapien mit starken elektromagnetischen Quellen, wie z.B. der Magnet-Resonanz-Tomographie, bei der der Einsatz einer Oximeteranordnung zur Kontrolle der Lebensfunktionen narkosierter Patienten im Magnet-Resonanz-Tomographen eine besonders Wichtige Rolle spielt, ist es überaus wichtig, eine Anordnung zu verwenden, die zum einen das homogene elektromagnetische Feld des Tomographen nicht stört und zum anderen Gefährdungen des Patienten, wie Verbrennungen oder Stromschläge, die durch Induktion in z.B. elektrische Zuleitungen entstehen können, ausschließt. Derartigen Anforderungen wird diese bekannte Oximeteranordnung nicht gerecht.

Der vorliegenden Erfindung liegt die Aufgabe zugrunde, eine nichtinvasive Oximeteranordnung mit einem klammerartigen Sensor für beispielsweise einen Finger zu schaffen, die zur Erfassung des Blutsauerstoffsättigungsgrades geeignet ist, ferner verträglich ist mit der elektromagnetisch beeinflußten Umgebung, z.B. des Inneren eines Magnet-Resonanz-Tomographen und hinsichtlich sonstiger Stör- und Fehlerquellen robust ist.

Diese Aufgabe Wird erfindungsgemäß dadurch gelöst, daß der Sensor und die Übertragungsstrecke aus nicht metallischem Material bestehen, der Sensor eine Federvorrichtung und mindestens eine Mulde mit einem einer Fingerkuppe angeformten Endabschnitt aufweist, in dem Lichtleiterabschnitte enden und lösbar zur bewegungsunempfindlichen Verbindung des Fingers mit letztlich den sensorseitigen Enden der Übertragungsstrecke verbunden sind, die zwei Lichtwellenleiter mit unterschiedlichem Querschnitt aufweist, und daß die Steuereinheit einen Sender, einen Empfänger und eine Recheneinheit zur Ermittlung des Sauerstoffgehalts des Blutes aus der relativen Größe des pulsschlagabhängigen Modulationsgrades der vom Finger reflektierten unterschiedlich kodierten, den spektralen Fenstern der Übertraungsstrecke entsprechenden elektromagnetischen Wellen aufweist.

Gemäß einer bevorzugten Ausgestaltung der Erfindung leitet der erste Leiter die elektromagnetischen wellen, nachfolgend auch kurz Wellen genannt, von der Steuereinheit zum Sensor und der zweite Leiter die letztlich vom Finger reflektierten elektromagnetischen Wellen zur Steuereinheit zurück, wobei der zweite Leiter einen größeren Querschnitt als der erste Leiter aufweist, beide aus Kunststoff sind und im Sensor über eine Steckvorrichtung mit den Lichtleiterabschnitten verbunden sind. Durch die Anordnung derartiger im Sensor fest angeordneter Lichtleiterabschnitte werden bereits Bewegungsfehler vermieden, die aus Relativbewegungen beider Leiter der Übertragungsstrecke während des Betriebes herrühren.

Vorteilhaft kann weiter vorgesehen sein, daß der Sensor ein Oberteil und ein Unterteil mit je einer rinnenförmigen Mulde zum Aufnehmen eines Teils des Fingers aufweist, welche klammerartig und federnd gegeneinander beweglich sind. Vorteilhaft weist die untere Mulde den inneren an der Fingerkuppe angeformten Endabschnitt auf, in dem die Lichtleiterabschnitte mit ihren Querschnittsflächen bündig und in Abstand zueinander angeordnet sind. Der Abstand der Querschnittsflächen zueinander dient im wesentlichen dazu, optische Kurzschlüsse zu vermeiden.

Erfindungsgemäß sind die Querschnittsflächen in einem Winkel entsprechend der Krümmung des Endabschnittes zueinander angeordnet und die Lichtleiterabschnitte in einer Vergußmasse eingebettet. Sie verlaufen zur Fingerspitze hin teilweise gekrümmt. Die Mulden können aus flexiblem Material zur Anpassung an unterschiedliche Fingerformen bestehen. Durch die Anordnung beider Querschnittsflächen in einer der Mulden, nämlich der unteren, können wiederum Bewegungsfehler, die durch unbeabsichtigte Relativbewegungen der beiden Klammerteile zueinander entstehen, vermieden werden.

Die Federvorrichtung ist vorteilhaft ein den Finger belastendes um das Ober- und Unterteil des Sensors gelegtes Gummiband, oder ein federnder Schaumstoff, der mit gleicher wirkung zwischen entsprechendne Teilen des Sensors angeordnet sein kann.

Gemäß einer bevorzugten Ausgestaltung weist der Sender für die erste und zweite elektromagnetische Welle je eine LED (light emiting diode) auf, welche aus einem Block mit je einem unterschiedlich modulierten entsprechenden Signal gespeist werden. Jeder LED ist ein Lichtleiter zugeordnet, der eingangsseitig in ein optisches Koppelglied führt, an dessen Ausgang der erste Leiter der Übertragungsstrecke angeschlossen ist.

Der Empfänger ist vorteilhaft eingangsseitig mit dem zweiten Leiter der Übertragungsstrecke verbunden und weist einen Wandler zur Erzeugung eines entsprechenden elektrischen Signals auf, dem eine Demodulationsschaltung mit Gleichrichtern nachgeordnet ist, wodurch für jede der zwei reflektierten Wellenlängen ein demoduliertes, gleichgerichtetes Ausgangssignal Vorliegt. Die Modulation der elektromagnetischen wellen dient u.a. zur Erkennung der vom Sender erzeugten zwei elektromagnetischen Wellen und somit auch der Unterdrückung von Störungen durch Fremdlicht.

Die Ausgangssignale des Empfängers werden eingangsseitig der Recheneinheit zugeführt. Diese weist erfindungsgemäß zwei Zweige auf, mit je einem Hochpaß mit einer nachgeordneten Schaltung zur Ermittlung der pulsabhängigen Amplitude, also des pulsschlagabhängigen Modulationsgrades, einem dazu parallelen Tiefpaß und einem ersten Glied zur Division des Ausgangssignals der genannten Schaltung durch das des Tiefpasses. Diesen beiden Gliedern zur Division ist ein weiteres Glied zur Division nachgeordnet, an dessen Ausgang ein Signal zur Weiterleitung an eine Anzeige oder Datenschnittstelle ansteht. Das Signal kann an eine Anzeigeeinhieit und/oder Datenschnittstelle weitergegeben werden.

Erfindungsgemäß wird eine untere erste Wellenlänge von 660 nm und eine obere zweite Wellenlänge von ca. 780 nm bis ca. 850 nm zur Nutzung der spektralen Fenster verwendet, wobei die erste Wellenlänge mit einer ersten Freguenz und die zweite Wellenlänge mit einer zweiten Frequenz vorzugsweise amplitudenmoduliert ist. Gegenüber einer bekannten ersten Wellenlänge von 660 nm und einer zweiten Wellenlänge von 805 nm ist erfindungsgemäß für die zweite ein wesentlich größerer Bereich möglich.

Gemäß einer bevorzugten Ausgestaltung kann aber auch vorgesehen sein, daß die erste und zweite Wellenlänge mit Hilfe eines Zeitmultiplexverfahrens moduliert sind.

Vorzugsweise wird eine Wellenlänge von ca. 830 nm für die zweite Wellenlänge verwendet. Da sich gezeigt hat, daß Wellenlängen von mehr als ca. 850 nm in den verwendeten Lichtleitfasern erheblich gedämpft werden, sind die Wellenlängen nach oben begrenzt.

Vorzugsweise sind alle Lichtleiter ein- bzw. mehradrig aufgebaut, wobei der erste Lieter eine Faser von z.B. 1 mm und der zweite Leiter, wegen hoher Lichtverluste im Finger, 32 Fasern mit z.B. je 0,5 mm Durchmesser aufweist.

Die erfindungsgemäße Oximeteranordnung benutzt somit zur Erfassung des Blutsauerstoffgehalts ein pulsförmiges Signal, das einem hohen Gleichsignaluntergrund überlagert ist und vom Blutvolumen im Finger im Rhythmus des Herzschlages abhängt. Der Modulationsgrad dieses kleinen Signals, d.h. der Quotient aus pulsförmiger Wechselsignalamplitude und Untergrund ist gleich dem produkt aus spezifischer Absorption des Blutes bei der verwendeten Wellenlänge multipliziert mit dem Blutvolumenpuls, bzw. der durch ihn bedingten Änderung der Länge des Lichtweges im Blutvolumen. Dieser Quotient hängt nicht mehr von der Gewebeabsorption und vom Koppelfaktor zwischen den Querschnittsflächen der Fasern und der Hautoberfläche ab. Der Quotient aus den auf ihren Untergrund normierten optischen Pulssignalen bei den o.g. Wellenlängen eliminiert letztlich noch die unbekannte Größe des Blutvolumens je Pulsschlag, so daß als Maß für den Sauerstoffgehalt der Quotient aus spezifischer Absorption des Blutes bei beiden verwendeten Wellenlängen am Ausgang der Recheneinheit zur Verfügung steht.

Weitere bevorzugte Ausgestaltungen der Erfindung ergeben sich aus den Unteransprüchen.

Ein Ausführungsbeispiel wird nachfolgend unter Bezugnahme auf die Zeichnung näher erläutert. Es zeigt:

Fig. 1 eine erfindungsgemäße nichtinvasive Oximeteranordnung, bei der der Sensor entlang seiner Längsachse geschnitten dargestellt ist,

Fig. 2 den prinzipiellen Aufbau einer Steuereinheit der Oximeteranordnung,

Fig. 3 eine typische Signalform einer reflektierten elektromagnetischen Welle am Ausgang eines Empfängers der Steuereinheit,

Fig. 4 den Sensor der Oximeteranordnung gemäß Fig. 1 in geöffneter Stellung, und

Fig. 5 eine Schnittdarstellung entlang der Linie A-A des Sensors der Oximeteranordnung gemäß Fig. 1.

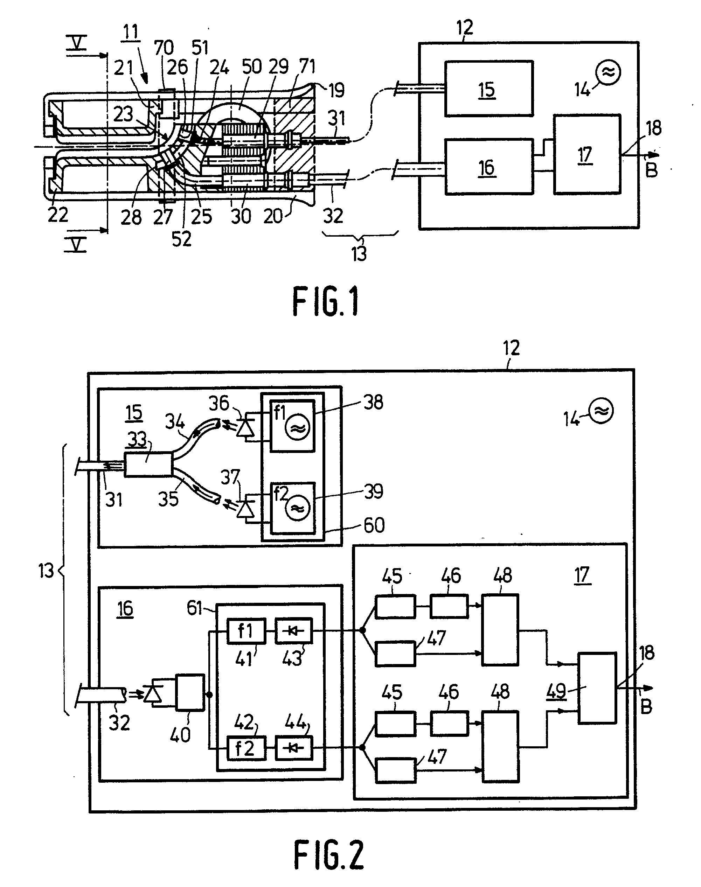

Fig. 1 zeigt eine erfindungsgemäße nichtinvasive Oximeteranordnung 10. Sie besteht aus einem klammerartigen Sensor 11, einer Steuereinheit 12 und aus einer vorzugsweise Zwei Lichtleiter 31, 32 umfassenden Übertragungsstrecke 13, die die Steuereinheit 12 mit dem Sensor 11 verbindet. Die Steuereinheit 12 Weißt eine Energieversorgung 14, einen Sender 15, einen Empfänger 16 und eine Recheneinheit 17 auf, an dessen Ausgang 18 das den Sauerstoffgehalt des Blutes repräsentierende Signal ansteht. Dieses Signal kann innerhalb der Steuereinheit 12 über eine nicht dargestellte Anzeige sichtbar gemacht werden oder aber beispielsweise einer ebenfalls nicht dargestellten Datenschnittstelle zugeführt werden.

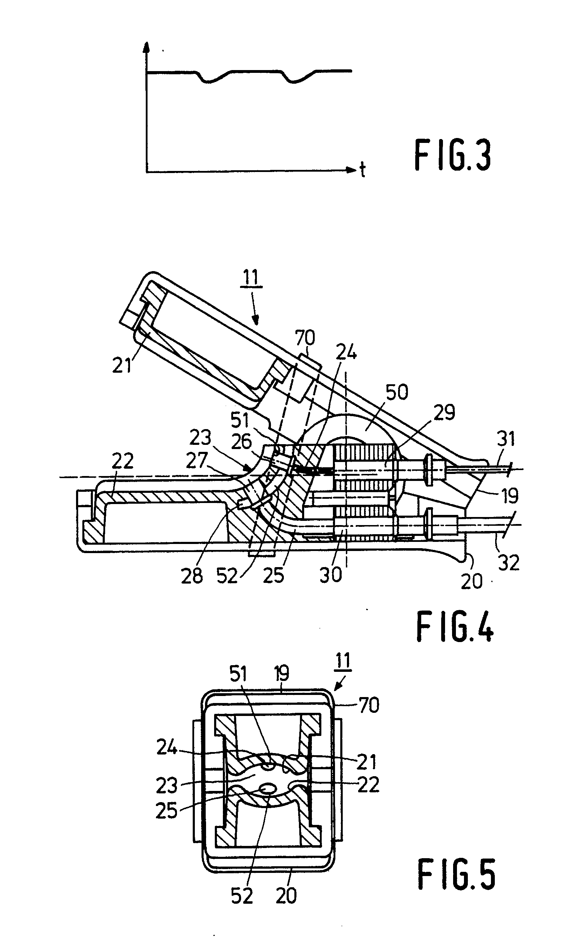

Der Sensor 11 ist klammerartig ausgestaltet und besteht im wesentlichen aus einem Oberteil 19 und einem Unterteil 20, die über ein Gelenk 50 miteinander beweglich verbunden sind. Der Sensor 11 ist vollständig aus nicht metallischem Material gefertigt und verharrt ohne eine äußere Hebel kraft mittels einer nicht metallischen Federvorrichtung aus einem um das Ober- und Unterteil 19, 20 gelegten Gummiband 70, oder aus einem federnden Schaumstoff 71, der zwischen die Teile 19 und 20 gelegt ist, im zugeschnappten Zustand. In Fig. 1 sind beide Möglichkeiten angedeutet und in Fig. 4 und 5 nur das Gummiband 70. Das Oberteil 19 und das Unterteil 20 weisen als Klemmbacken rinnenförmig ausgeformte Mulden 21 und 22 auf. Diese Mulden 21 und 22 bilden ein Fingerbett aus vorzugsweise, weichem elastischem Kunststoffmaterial, in das zumindest ein Teil, vorzugsweise zwei Glieder eines nicht dargestellten Zeigefingers sozusagen eingespannt werden können. Die untere Mulde 22 ist in den klammerartigen Sensor weisend durch einen Endabschnitt 23 begrenzt, der seiner Form nach geeignet ist, die Fingerkuppe des Fingers bündig abzustützen. In der Oberfläche dieses Endabschnittes 23 sind zum Finger weisend bündig die Querschnittsflächen 51, 52 von zwei Lichtleiterabschnitten 24 und 25 in Abstand zueinander angeordnet. Zum Endabschnitt 23 hin können Endstücke 26 und 27 zur Aufnahme der Enden der Lichtleiterabschnitte 24 und 25 vorgesehen sein, die ihrerseits wiederum mittels einer Vergußmasse 28 festgesetzt sind. Die Lichtleiterabschnitte 24 und 25 führen dann leicht gekrümmt zu je einer Steckvorrichtung 29 und 30 des Unterteils 20 des Sensors 11.

Die Lichtleiterabschnitte 24 und 25 sind ebenso wie die Leiter 31 und 32 der Übertragungsstrecke 13 kommerzielle Kunststofflichtwellenleiter. Leiter 31 bildet eine Sendeleitung hinsichtlich der Steuereinheit 12 und Leiter 32 eine Empfangsleitung. Als Sendeleitung Wird beispielsweise eine Faser mit 1 mm Durchmesser benutzt. Der hohe Lichtverlust beim Durchstrahlen des Fingers macht es notwendig, für die Empfangsleitung einen größeren Querschnitt vorzusehen. Es kann dazu ein Lichtwellenleiterbündel mit 32 Fasern von je 0,5 mm Durchmesser eingesetzt werden. Alternativ läßt sich selbstverständlich auch eine Faser mit beispielsweise 3 mm Durchmesser verwenden, die dann allerdings sehr starr ist. Die Leiter 31 und 32 sind über die Stecker 29 und 30 mit dem Sensor 11 verbunden. Die über den Leiter 31 im Sensor 11 ankommenden elektromagnetischen Wellen treten im Endabschnitt 23 aus und in die Fingerkuppe ein. Dadurch, daß die Querschnittsfläche des Lichtleiterabschnittes 25 ebenfalls im Endabschnitt 23 endet, weist sie einen winkel gegenüber der im Endabschnitt 23 endenden Querschnittsfläche 51 des Lichtleiterabschnittes 24 auf. Ein Teil der vom Finger reflektierten elektromagnetischen Wellen gelangt dann in den Lichtleiterabschnitt 25 und über die Steckvorrichtung 30 in den Leiter 32 und somit in die Steuereinheit 12.

Durch diese Anordnung wird der Finger nicht vollkommen durchstrahlt. Die Lichteinkopplung und -auskopplung Werden vielmehr beide an der Fingerkuppe vorgenommen, weshalb beide Lichtleiter starr Von einer Seite an den Finger herangeführt werden können, wodurch Bewegungsfehler, die beispielsweise über eine veränderte optische Kopplung bei Kabelbewegungen hervorgerufen werden, reduziert sind.

Fig. 2 Zeigt eine Steuereinheit 12, in der der prinzipielle Aufbau des Senders 15, des Empfängers 16 und der Recheneinheit 17 dargestellt ist.

Der Sender 15 weist im wesentlichen einen optischen Koppler 33 auf, der ausgangsseitig mit dem Leiter 31 und eingangsseitig mit zwei weiteren Lichtleitern 34 und 35 verbunden ist. Die Lichtleiter 34 und 35 führen zu je einer LED 36 und 37, die aus einem Block 60 mit je einem elektrischen Signal versorgt werden. Der Block 60 kann dazu je einen Generator 38 und 39 beinhalten. Der Generator 38 erzeugt ein Signal zur Erzeugung einer elektromagnetischen Welle von 660 nm, wobei das Signal vorzugsweise mit einer Frequenz $f_1$ amplitudenmoduliert ist. Der Generator 39 erzeugt ein Signal zur Erzeugung einer elektromagnetischen welle von vorzugsweise 830 nm, wobei das Signal mit einer Frequenz $f_2$ amplitudenmoduliert ist. Hier sei darauf hingewiesen, daß im Block 60 auch eine andere in der Fig. 2 nicht näher ausgeführte Modulation, z.B. eine Zeit-Multiplex-Modulation vorgesehen sein kann. Als zweite Wellenlänge kann auch Licht mit einer Wellenlänge von ca. 780 nm bis ca. 850 nm verwendet werden. Größere Wellenlängen werden in den Kunststoffasern zu stark gedämpft, weshalb sie weniger geeignet sind.

Die zur Vermeidung von Fremdlichteinflüssen auf das Meßergebnis bezüglich ihrer Intensität modulierten elektromagnetischen Wellen gelangen nach Reflexion im Finger letztlich über Leiter 32 in den Empfänger 16 und dort auf einen Wandler 40 zur Erzeugung von entsprechenden elektrischen Signalen. Die Selektion der am Ausgang des Wandlers 40 vorhandenen Meßinformation geschieht in einer Demodulationsschaltung 61, im Fall der Amplitudenmodulation gemäß Fig. 2 mit je einem nachgeordneten Filter 41 und 42 entsprechend den Modulationsfrequenzen $f_1$ und $f_2$ und einer nachfolgenden Gleichrichtung in den Gleichrichtern 43 und 44. Nach der Demodulation stehen am Ausgang des Empfängers 16 zwei Signale zur Verfügung, die jeweils proportional zur optischen Reflexion des Fingers im Sensor 11 sind. Hier sei angemerkt, daß Taktsignale vom Sender 15 an die Demodulationsschaltung 61 übergeben Werden.

In Fig. 3 ist die Form der Signale am Ausgang des Empfängers 16 dargestellt. Die Form besteht im wesent lichen aus einem hohen Signaluntergrund auf dem kleine Signaländerungen im Herzschlagrhythmus zu beobachten sind. Diese stammen vom Pulsschlag im durchstrahlten Finger. Dieses kleine pulsförmige Signal Wird zur Erfassung des Blutsauerstoffgehalts herangezogen, da es der durch die Herztätigkeit bedingten Änderung des Blutvolumens, also dem Blutvolumenpuls entspricht, somit hauptsächlich aus dem arteriellen Bereich des Blutkreislaufs stammt und daher ein bevorzugter Informationsträger für den Sauerstoffsättigungsgrad ist. Der Modulationsgrad dieses kleinen Signals, d.h. der Quotient aus pulsförmiger Wechselamplitude und Untergrund ist gleich dem Produkt aus spezifischer Absorption des Blutes bei der verwendeten Wellenlänge multipliziert mit dem Blutvolumenpuls, bzw. der durch ihn bedingten Änderung der Länge des Lichtweges im Blutvolumen. Dieser Quotient hängt von keiner der eingangs genannten Störgrößen mehr ab. Er ist zudem unabhängig von der Primärintensität der Lichtquelle, den Dämpfungsverlusten auf den Lichtleitfaserstrecken sowie den Eigenschaften der Fotodiode und des Wandlers selbst. Der Quotient aus den auf ihren Untergrund normierten optischen Pulssignalen bei den o.g. Wellenlängen eliminiert letztlich noch die unbekannte Größe des Volumenpulses. Als Ergebnis bleibt der Quotient aus spezifischer Absorption des Blutes bei beiden verwendeten Wellenlängen über und ist ein Maß für den Sauerstoffgehalt.

In der Recheneinheit 17, der die Ausgangssignale des Empfängers 16 als Eingangssignale zugeführt sind, wird dieser Quotient, der den Sauerstoffgehalt repräsentiert, ermittelt und als Ausgangssignal an eine nicht dargestellte Anzeige und/oder an eine Datenschnittstelle entsprechend Pfeil B weitergegeben. Jedem Eingangssignal der Recheneinheit ist ein Zweig zugeordnet, bestehend aus einem Hochpaßfilter 45, einer nachgeordneten Schaltung 46 zur Ermittlung der Pulsabhängigen Amplitude, einem dazu Parallelgeschalteten Tiefpaßfilter 47 und einem Glied 48, das mit den Ausgängen der Schaltung 46 und des Tiefpaßfilters 47 verbunden ist. Jedes Glied 48 dient der Quotientenbildung zur Berechnung der relativen Pulshöhe für jedes der beiden demodulierten Signale, also der Division der Höhe des pulsförmigen Signals, das durch den Hochpaßfilter 45 mit der nachgeordneten Schaltung 46 gebildet wird, durch den hohen Signaluntergrund, der am Ausgang des Tiefpaßfilters 47 ansteht. Die Ausgangssignale der beiden Glieder 48 werden eingangsseitig einem weiteren Glied 49 zugeführt. Dieses Glied 49 ist ein Dividierer und dient dazu, eine Division der beiden den relativen Pulshöhen proportionalen Eingangssignale durchzuführen, um am Ausgang das gewünschte nur noch vom Sauerstoffgehalt des Blutes abhängige Signal zu erzeugen.

Die Schaltung 46 kann als Spitzenwertgleichrichter ausgebildet sein, der bei kleinen Signalen jedoch störanfällig sein kann. Vorteilhaft ist die Verwendung einer Schaltung 46 mit einer Komparatorschaltung zur Ermittlung des Pulsschlages bzw. eines entsprechenden Triggerimpulses und einem Minimum-Maximum-Detektor mit einem nachgeordneten Speicherglied. Gemäß einer vorteilhaften Ausgestaltung kann nur eine Komparatorschaltung im Zweig mit der größeren Wellenlänge vorgesehen sein, die dann den Minimum-Maximum-Detektor im Zweig mit der kleineren Wellenlänge ebenfalls mit dem herzschlagabhängigen Triggerimpuls versorgt.

Fig. 4 Zeigt den Sensor 11 aus Fig. 1 in geöffneter Stellung und Fig. 5 eine Schnittdarstellung entlang der Linie A-A gemäß Fig. 1 durch den Sensor 11. In dieser Darstellung sind die rinnenförmigen Mulden 21 und 22 als auch die Querschnittsflächen 51 und 52 der im Endabschnitt 23 endenden Leiterabschnitte 24 und 25 deutlich zu erkennen.

Die in der vorstehenden Beschreibung, in den Figuren sowie in den Ansprüchen offenbarten Merkmale der Erfindung können sowohl einzeln als auch in beliebiger Kombination für die Verwirklichung der Erfindung in ihren verschiedenen Ausführungsformen wesentlich sein.

**Ansprüche**

1. Nichtinvasive Oximeteranordnung mit einem klammerartigen Sensor für beispielsweise einen Finger, einer selbstkalibrierenden Steuereinheit zur Erzeugung und Verarbeitung von zwei elektromagnetischen Wellen bestimmter Wellenlänge und einer zwischengeschalteten Übertragungsstrecke, dadurch gekennzeichnet, daß der Sensor (11) und die Übertragungsstrecke (13) aus nicht metallischem Material bestehen, der Sensor (11) eine Federvorrichtung und mindestens eine Mulde (22) mit einem einer Fingerkuppe angeformten Endabschnitt (23) aufweist, in dem Lichtleiterabschnitte enden und lösbar zur bewegungsunempfindlichen Verbindung des Fingers mit letztlich den sensorseitigen Enden der Übertragungsstrecke (13) verbunden sind, die zwei Lichtwellenleiter (31, 32) mit unterschiedlichem Querschnitt aufweist, und daß die Steuereinheit (12) einen Sender (12), einen Empfänger (16) und eine Recheneinheit (17) zur Ermittlung des Sauerstoffgehalts des Blutes aus der relativen Größe des pulsschlagabhängigen Modulationsgrades der vom Finger reflektierten zuvor unterschiedlich kodierten, den spektralen Fenstern der Übertragungsstrecke (13) entsprechenden elektromagnetischen Wellen aufweist.

2. Anordnung nach Anspruch 1, dadurch gekennzeichnet, daß der erste Leiter (31) die elektromagnetischen Wellen von der Steuereinheit (12) zum Sensor (11) und der zweite Leiter (32) die letztlich vom Finger reflektierten elektromagnetischen Wellen zur Steuereinheit (12) zurückleitet, daß der erste Leiter (31) einen geringeren Querschnitt als der zweite Leiter (32) aufweist, beide aus Kunststoff sind und im Sensor (11) über Steckvorrichtungen (29, 30) mit den Lichtleiterabschnitten (24, 25) verbunden sind.

3. Anordnung nach Anspruch 2, dadurch gekennzeichnet, daß der Sensor (11) ein Oberteil (19) und ein Unterteil (20) mit je einer rinnenförmigen Mulde (21, 22) zur Bildung eines Fingerbettes für einen Teil des Fingers aufweist, welche klammerartig federnd gegeneinander beweglich sind, daß die untere Mulde (22) den inneren Endabschnitt (23) aufweist, in dem die Lichtleiterabschnitte (24, 25) mit ihren Querschnittsflächen (51, 52) bündig und in Abstand zueinander angeordnet sind.

4. Anordnung nach Anspruch 3, dadurch gekennzeichnet, daß die Querschnittsflächen (51, 52) in einem Winkel entsprechend der Krümmung des Endabschnitts (23) zueinander angeordnet sind, daß die Lichtleiterabschnitte (24, 25) in einer Vergußmasse (28) eingebettet sind und zum Endabschnitt (23) hin gekrümmt verlaufen, und daß die Mulden (21, 22) aus flexiblem Material zur Anpassung an unterschiedliche Fingerformen bestehen.

5. Anordnung nach Anspruch 4, dadurch gekennzeichnet, daß die Federvorrichtung ein den Finger belastendes um das Ober- und Unterteil (19, 20) gelegtes Gummiband (70) und/oder ein zwischen den Teilen (19, 20) mit gleicher Wirkung angeordneter federnder Schaumstoff (71) ist.

6. Anordnung nach Anspruch 5, dadurch gekennzeichnet, daß der Sender (15) für die erste und zweite elektromagnetische Welle je eine LED (36, 37) aufweist, welche aus einem Block (60) mit je einem unterschiedlich modulierten entsprechenden Signal gespeist werden, daß jeder LED (36, 37) ein Lichtleiter (34, 35) zugeordnet ist, der eingangsseitig in ein optisches Koppelglied (33) führt, an dessen Ausgang der erste Leiter (31) der übertragungsstrecke (13) angeordnet ist.

7. Anordnung nach Anspruch 6,

dadurch gekennzeichnet, daß der Empfänger (16) eingangsseitig mit dem zweiten Leiter (32) verbunden ist, ein nachgeordneter Wandler (40) ein entsprechendes elektrisches Signal erzeugt, dem eine Demodulationsschaltung (61) mit Gleichrichtern (43, 44) nachgeordnet ist, wodurch für jede der reflektierten zwei Wellenlängen ein demoduliertes, gleichgerichtetes Ausgangssignal am Empfänger (16) vorliegt.

8. Anordnung nach Anspruch 7,
dadurch gekennzeichnet, daß die Recheneinheit (17) zwei Zweige aufweist, die mit je einem Ausgangssignal des Empfängers (16) Verbunden sind und je einen Hochpaßfilter (45) mit einer nachgeordneten Schaltung (46) zur Ermittlung der pulsabhängigen Amplitude, einen dazu parallelgeschalteten Tiefpaßfilter (47) und ein nachgeordnetes Glied (48) zur Division des Ausgangssignals der Schaltung (46) durch das des Tiefpaßfilters (47) beinhalten, und ferner ein weiteres den beiden Gliedern (48) nachgeordnetes Glied (49) zur Division vorhanden ist, an dessen Ausgang das der Sauerstoffsättigung entsprechende Signal ansteht und an eine Anzeigeeinheit und/oder Datenschnittstelle weitergegeben wird.

9. Anordnung nach Anspruch 8,
dadurch gekennzeichnet, daß die Schaltung (46) einen Spitzenwertgleichrichter enthält.

10. Anordnung nach Anspruch 8,
dadurch gekennzeichnet, daß die Schaltung (46) eine Reihenschaltung aus einem Minimum-Maximum-Dekoder und einem Speicherglied aufweist, ferner eingangsseitig eine Komparatorschaltung, die mit dem Minimum-Maximum-Dekoder und dem Speicherglied zur Übergabe eines Pulsabhängigen Triggerimpulses verbunden ist.

11. Anordnung nach Anspruch 10,
dadurch gekennzeichnet, daß nur die Schaltung (46) im Zweig mit der größeren Wellenlänge eine Komparatorschaltung aufweist und diese die Triggerimpulse auch an die andere Schaltung (46) abgibt.

12. Anordnung nach einem oder mehreren der Vorhergehenden Ansprüche,
dadurch gekennzeichnet, daß eine untere erste wellenlänge von ca. 660 nm und eine obere zweite Wellenlänge von ca. 780 nm bis Ca. 804 nm und ca. 806 nm bis ca. 850 nm zur Nutzung der spektralen Fenster verwandt wird, wobei die erste Wellenlänge mit einer ersten Frequenz $f_1$ und die zweite Wellenlänge mit einer zweiten Frequenz $f_2$ über Generatoren (38, 39) des Blockes (60) amplitudenmoduliert ist, und daß die Demodulationsschaltung (61) aus je einer Reihenschaltung aus einem Filter (41; 42) und einem der Gleichrichter (43; 44) aufgebaut ist.

13. Anordnung nach Anspruch 12,
dadurch gekennzeichnet, daß eine untere erste Wellenlänge von ca. 660 nm und eine obere zweite Wellenlänge von ca. 780 nm bis ca. 804 nm und ca. 806 nm bis ca. 850 nm zur Nutzung der spektralen Fenster verwendet wird, wobei beide Wellenlängen mit Hilfe eines Multiplexverfahrens über den Block (60) moduliert und in der Demodulationsschaltung (61) demoduliert sind.

14. Anordnung nach einem oder mehreren der vorhergehenden Ansprüche,
dadurch gekennzeichnet, daß die zweite Wellenlänge ca. 830 nm beträgt.

15. Anordnung nach einem oder mehreren der vorhergehenden Ansprüche,
dadurch gekennzeichnet, daß alle Lichtleiter ein- bzw. mehradrig aufgebaut sind und der erste Leiter (31) eine Faser mit 1 mm und der zweite Leiter (32), wegen hoher Lichtverluste im Finger, 32 Fasern mit je 0,5 mm Durchmesser aufweist.

EP 0 430 340 A2

FIG.1

FIG.2

9

FIG.3

FIG.4

FIG.5